(19) Europäisches Patentamt
European Patent Office
Office européen des brevets

(11) Numéro de publication : **0 068 983**
**B1**

(12) **FASCICULE DE BREVET EUROPÉEN**

(45) Date de publication du fascicule du brevet : **26.03.86**

(51) Int. Cl.[4] : **C 07 D333/20**// C07F9/40

(21) Numéro de dépôt : **82401101.9**

(22) Date de dépôt : **17.06.82**

(54) **Procédé de préparation de dérivés des (thiényl-2)- et (thiényl-3)-2 éthylamines.**

(30) Priorité : **30.06.81 FR 8113064**

(43) Date de publication de la demande : **05.01.83 Bulletin 83/01**

(45) Mention de la délivrance du brevet : **26.03.86 Bulletin 86/13**

(84) Etats contractants désignés : **AT BE CH DE FR GB IT LI LU NL SE**

(56) Documents cités :
**JUSTUS LIEBIGS: "Annalen der chemie", vol.686, 1965, Verlag Chemie GmbH, Weinheim (DE), H. ZIMMER et al.: "Synthesen mit alpha-amino-alkyl-phosphonsäureestern, I", pages 107-114**
**TETRAHEDRON LETTERS, no.26, 1979, Pergamon Press Ltd., (GB), N.L.J.M. BROEKHOF et al.: "Enamine synthesis by the horner-wittig-reaction", pages 2433-2436**

(73) Titulaire : **SANOFI, Société dite: 40, Avenue George V F-75008 Paris (FR)**

(72) Inventeur : **Chekroun, Isaac 26 Rue Peyras F-31000 Toulouse (FR)**
Inventeur : **Heymes, Alain Chemin de l'Adrech F-04200 Sisteron (FR)**

(74) Mandataire : **Polus, Camille et al c/o Cabinet Lavoix 2, Place d'Estienne d'Orves F-75441 Paris Cedex 09 (FR)**

**EP 0 068 983 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à un nouveau procédé de préparation de thiénylamines répondant à la formule I

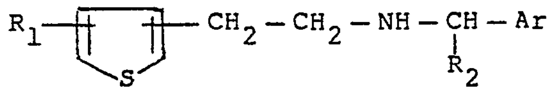

(I)

dans laquelle

$R_1$ (en position 2, 3, 4 ou 5) représente un atome d'hydrogène, un radical alkyle linéaire ou ramifié, un radical aromatique hétérocyclique ou non (en particulier thiényle, furyle, pyridyle, phényle, naphtyle) éventuellement mono- ou polysubstitué par des goupes alkyle, phényle, halogène, nitro, cyano, amino, carboxy ou alcoxy, ou bien un radical alcoxy, un atome d'halogène, un radical nitro, carboxyle, cyano ou amino ;

dans la chaîne aminoéthyle qui peut occuper les positions 2 ou 3 du noyau thiophénique, $R_2$ signifie un atome d'hydrogène ou un radical alkyle linéaire ou ramifié, un radical aromatique hétérocyclique ou non (en particulier thiényle, furyle, pyridyle, phényle, naphtyle) éventuellement mono- ou polysubstitué par des groupes alkyle, phényle, halogène, nitro, cyano, amino, carboxy ou alcoxy ;

Ar représente un radical aromatique tel que décrit ci-dessus pour $R_1$ et $R_2$.

Un certain nombre de dérivés répondant à la formule I sont connus et utilisés comme intermédiaires dans la préparation de composés employés aussi bien dans l'industrie chimique que pharmaceutique.

C'est ainsi, qu'à titre d'exemple, on peut mentionner parmi les dérivés obtenus selon le nouveau procédé de préparation ceux qui peuvent conduire par des moyens connus d'une part (la chaîne amino-éthyle étant en 2 et le radical $R_1$ en 4 ou 5) aux dérivés de la trétrahydro 4,5,6,7 thiéno [3,2-c] pyridine d'autre part (la chaîne amino-éthyle étant en 3 et le radical $R_1$ en 4 ou 5) à ceux de la tétrahydro 4,5,6,7 thiéno [2,3-c] pyridine, dérivés qui dans les deux cas ont fait l'objet de la part de la présente Demanderesse pour leurs applications en thérapeutique et/ou leurs procédés de préparation de plusieurs brevets (FR-2 215 948, FR-2 300 090, FR-2 315 274, FR-2 319 642, FR-2 345 150, FR-2 336 932, FR-2 397 417).

L'invention a pour objet un procédé simple et peu coûteux, en regard de la technique antérieure, d'obtention des composés de formule I.

Conformément au procédé de l'invention, pour préparer les dérivés de formule I :

a) on condense un dérivé de formule II

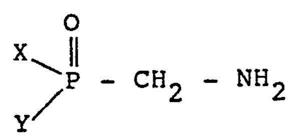

(II)

dans laquelle X et Y représentent ensemble ou séparément un radical alkyle, aryle, alkoxy, aryloxy, dialkyl- ou diarylamino (de telle sorte que le composé organophosphoré de formule II peut être par exemple un phosphonate, un phosphinate, un oxyde de phosphine ou un phosphonamide), avec un composé carbonylé de formule III

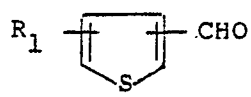

(III)

dans laquelle $R_1$ est tel que défini pour la formule I, pour conduire à un composé de formule IV

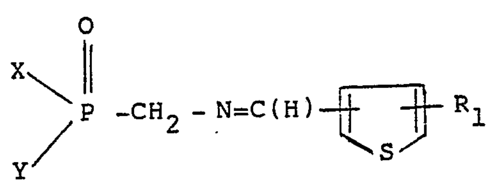

(IV)

dans laquelle $R_1$, X et Y ont les significations fournies précédemment ;

b) le composé de formule IV est traité avec une base de formule $B^{\ominus} M^{\oplus}$ dans laquelle $M^{\oplus}$ est un métal alcalin ou alcalino-terreux ou un organomagnésium et $B^{\ominus}$ est un carbanion choisi parmi les groupes chargés négativement hydrogène, hydroxy, amidure, mono- ou di-alkylamidure, alkyle linéaire ou ramifié, ou alcoxy linéaire ou ramifié, pour conduire à un carbanion de formule V

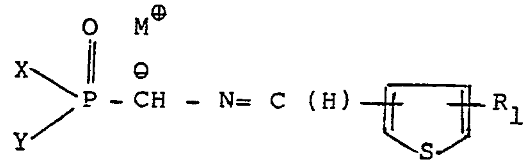

(V)

dans laquelle $R_1$, X, Y et $M^+$ ont les significations précédentes ;

c) qui se transforme à des températures comprises entre — 78 °C et + 150 °C en dérivé VI

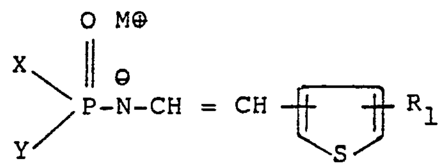

(VI)

dans laquelle $R_1$, X, Y et $M^+$ ont les significations précédentes, pour aboutir, après reprise par l'eau, au composé de formule VII

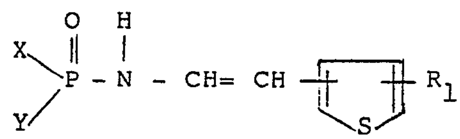

(VII)

dans laquelle $R_1$, X et Y ont les significations précédentes ;

d) le composé de formule VII est transformé sous l'action d'un agent réducteur en dérivé de formule VIII

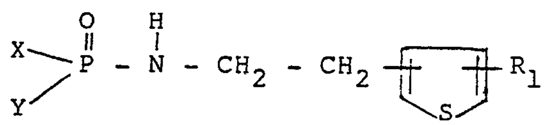

(VIII)

dans laquelle $R_1$, X et Y ont les significations précédentes ;

e) que l'on met à réagir successivement avec une base de formule B'⊖ M'⊕ dans laquelle $B'^-$ et M'+ ont respectivement les mêmes significations que $B^-$ et M+ ci-dessus, puis un dérivé halogéné de formule IX

$$Ar—CHX'—R_2 \qquad (IX)$$

dans laquelle Ar et $R_2$ sont tels que définis pour la formule I et X' représente un halogène, pour obtenir un dérivé de formule (X)

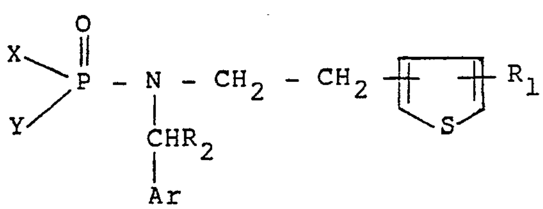

(X)

dans laquelle $R_1$, $R_2$, X, Y et Ar ont les significations précédentes ;

f) le dérivé de formule X est finalement transformé sous l'action d'un acide en composé de formule I tel que défini plus haut.

Le procédé de préparation suivant l'invention peut être illustré par le schéma réactionnel suivant :

a) 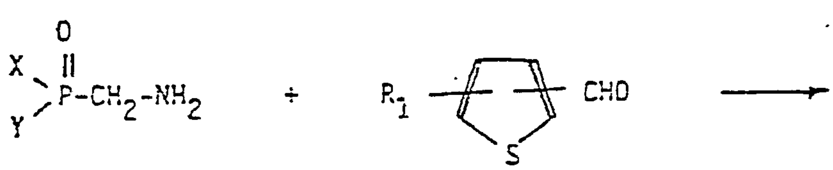

(II) (III)

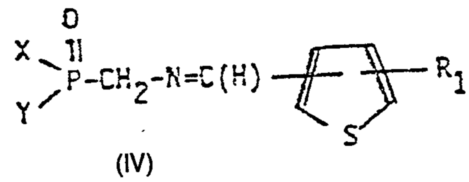

(IV)

b) (IV)

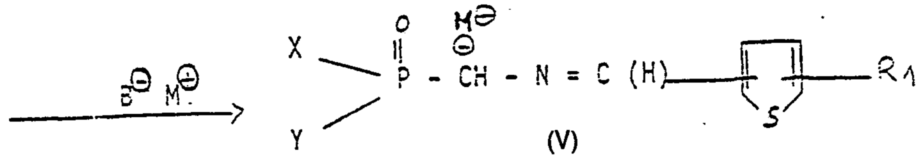


(V)

c) (V)

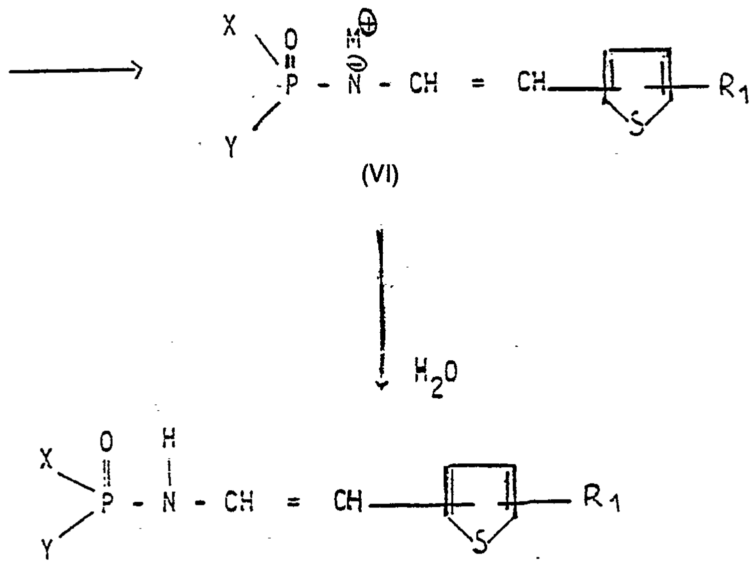


(VI)

(VII)

d) (VII)

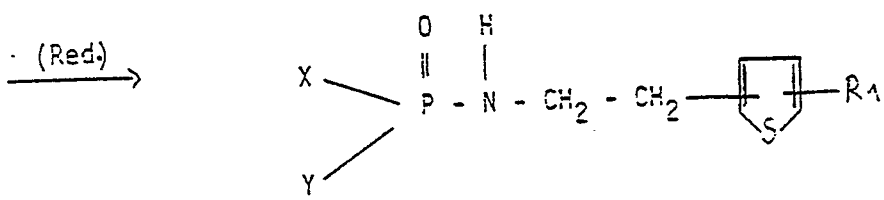


(VIII)

e) (VIII)

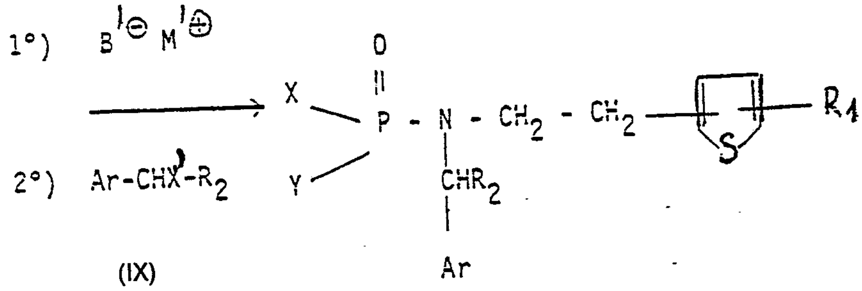


(IX)

(X)

(Suite)

f) (X) 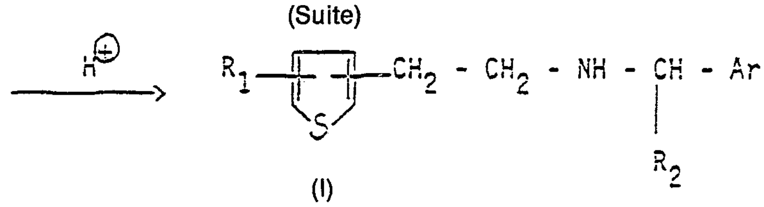


(I)

Selon une variante de l'invention, on inverse le déroulement des 5 stades d et e qui s'écrivent alors,

b) (IV)

d) (bis) (VI) 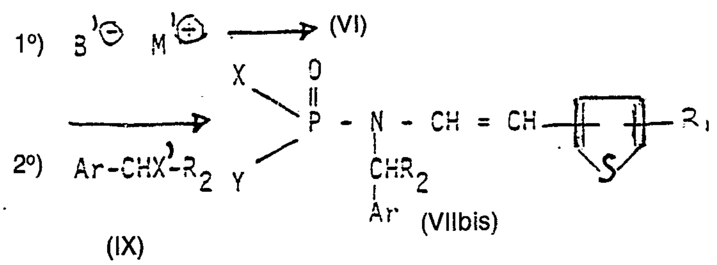


(VI)

(IX) (VIIbis)

e) (bis) (VIIIbis) 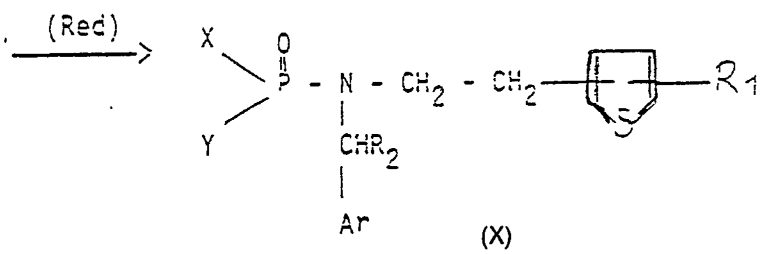


(X)

Le procédé peut avantageusement être mis en œuvre comme suit :

a) Les composés organophosphorés de formule II, facilement accessibles par des procédés d'obtention bien connus tel que celui par exemple décrit par I.C. POPOFF et Coll. (J. Org. Chem., *28,* 2898 (1963)) peuvent être mis à réagir avec les dérivés carbonylés III en l'absence de solvant et de catalyseur, l'eau formée au cours de la réaction étant éliminée à la fin de l'opération par des moyens appropriés. La condensation peut s'effectuer avantageusement dans un solvant tel qu'un hydrocarbure aromatique par exemple le toluène ou un alcool par exemple l'éthanol dans lesquels il est possible d'éliminer l'eau par distillation azéotropique. La condensation peut encore être avantageusement réalisée (sur le plan de la vitesse) en présence de quantités catalytiques d'un acide minéral ou organique comme par exemple l'acide paratoluènesulfonique. La température à laquelle on effectue cette transformation est variable mais se situe très généralement entre 20 et 120 °C.

b, c) La base B⊖ M⊕, mise en œuvre dans ce stade peut être un hydrure de métal alcalin, notamment les hydrures de sodium, de lithium ou de potassium, un amidure ou alkylamidure, notamment dialkylamidure, de métal alcalin tel que le diisopropylamidure de lithium, un composé organométallique, notamment les organolithiens tel que le n-butyl-lithium ou les organosodés ou les organomagnésiens. On peut aussi faire appel aux alcoolates de métal alcalin ou alcalino-terreux, tels que le méthylate de sodium, de lithium, de potassium, de magnésium, le tertiobutylate de potassium, le tertioamylate de sodium. On peut encore utiliser des hydroxydes de métal alcalin ou alcalino-terreux, tels que l'hydroxyde de sodium, de lithium, de potassium, de magnésium.

En général, on utilise un équivalent stœchiométrique de la base B⊖ M⊕, voire un léger excès par exemple de 10 % par rapport à l'équivalence. Mais, il est également possible de mettre en œuvre des quantités de base inférieures voire nettement inférieures à l'équivalence stœchiométrique.

Il convient de souligner que lorsqu'on opère selon la variante dans laquelle on inverse le déroulement des stades d et e et que l'on utilise un équivalent stœchiométrique de la base B⊖ M⊕, il peut être avantageux d'éviter l'isolement du dérivé VII et de mettre à réagir directement VII avec le dérivé halogéné IX en évitant de la sorte également l'utilisation de la base B'⊖ M'⊕.

On opère généralement entre — 78 °C et + 150 °C à une température choisie plus spécifiquement en fonction de la base B⊖ M⊕ dans une plage plutôt haut de gamme, en particulier lors de la réalisation de la phase c).

Les solvants préférés sont les éthers linéaires ou cycliques tels que le tétrahydrofuranne, les hydrocarbures, notamment les aromatiques tels que benzène, toluène, xylènes, les alcools, les amides notamment la diméthyl formamide, les sulfoxides notamment le diméthylsulfoxide. Il peut être également avantageux, particulièrement quand on utilise des hydroxydes métalliques, d'opérer en système

biphasique (eau + solvant tel que solvant halogéné comme par exemple le dichlorométhane ou hydrocarbure aromatique tels que benzène, toluène, xylènes) en présence d'un catalyseur de transfert de phase notamment un sel d'ammonium quaternaire tel que l'iodure de tétra-n-butylammonium ou un sel de phosphonium. Les traitements habituels permettent d'isoler le composé VII.

d) La réduction du dérivé de formule VII est effectuée avantageusement par un hydrure mixte de métal alcalin comme notamment un borohydrure tel que le borohydrure de sodium ou le borohydrure de potassium. La réduction est réalisée au sein d'un solvant inerte comme un éther tel que par exemple le tétrahydrofurane ou le dioxanne ou encore dans un alcool tel que par exemple le méthanol ou l'éthanol.

On peut également effectuer cette réduction par le biais d'une hydrogénation catalytique en phase homogène ou hétérogène dans des conditions bien connues d'une manière générale.

e) La base B'⊖ M'⊕, mise en œuvre dans la première partie de ce cinquième stade, peut être choisie parmi celles énumérées aux b) et c) ci-dessus. En général, elle est utilisée en équivalence stœchiométrique mais peut être en léger excès par exemple de 5 à 10 % par rapport à cette équivalence.

On opère généralement entre — 20 °C et + 100 °C avec une préférence pour le bas de gamme. Les solvants utilisés sont ceux décrits au stade b) ci-dessus.

Dans la seconde partie de ce stade, le composé halogéné de formule IX est mis à réagir avec le milieu réactionnel tel que défini ci-dessus à une température généralement similaire à celle de la première partie.

f) La coupure acido-catalysée de la liaison phosphore-azote du dérivé IX peut être réalisée en mettant en œuvre un acide minéral tel qu'un acide halohydrique comme par exemple l'acide chlorhydrique ou l'acide bromhydrique, mais également un acide organique en particulier un acide fort tel qu'un acide sulfonique comme par exemple l'acide benzènesulfonique ou l'acide paratoluènesulfonique. Les solvants préférés sont les éthers en particulier les éthers cycliques comme le tétrahydrofurane ou le dioxanne, les alcools comme le méthanol ou l'éthanol, les amides notammnet la diméthylformamide, les sulfoxides notamment le diméthylsulfoxide. Il est possible de travailler au sein de ces solvants en l'absence d'eau mais aussi dans des mélanges contenant des proportions variables de cette dernière. Il est enfin possible de travailler au sein de l'eau elle-même.

En général, on utilise un à deux équivalents stoechiométriques d'acide.

On opère généralement entre 0 et 100 °C et plus particulièrement entre 30 et 70 °C.

Les composés de formule I ainsi obtenus peuvent ensuite être isolés et purifiés selon les méthodes habituelles. Pour réaliser ces opérations il peut être avantageux de transformer les composés libres de formule I en leurs sels, par exemple en leurs sels d'addition d'acides par réaction avec des acides minéraux ou organiques. A partir des sels, on peut libérer les composés de formule I selon les méthodes connues.

Les exemples suivants illustrent l'invention sans la limiter.

Exemple 1

Préparation du chlorhydrate de N-orthochlorobenzyl, (thiényl-2)-2 éthylamine

Variante A

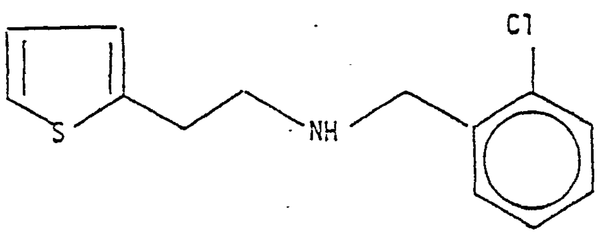

, HCl

stade a — N(thiénylidène-2), aminométhylphosphonate de diéthyle

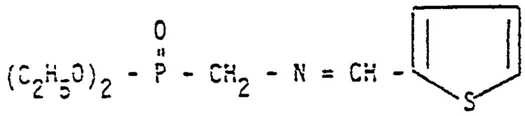

A 16,7 g (0,1 mole) d'aminométhylphosphonate de diéthyle dans 200 ml d'éthanol absolu, on ajoute 11,2 g (0,1 mole) de thénaldéhyde-2 et on porte 30 mm au reflux. L'eau formée au cours de la réaction est éliminée par distillation azéotropique. Après évaporation complète du solvant, on recueille 28 g (env. 100 %) d'une huile jaune pure par (CPL, CCM et CPG).

IR (film)
- C = N 1 645 $cm^{-1}$
- P = O 1 260 $cm^{-1}$
- P—O—C 1 060-1 080 $cm^{-1}$

RMN ($CDCL_3$) δ/TMS
- 1,3 ppm (t, 6H)
- 3,9 à 4,45 ppm (m, 6H)
- 7 à 7,6 ppm (m, 3H)
- 8,5 ppm (d, 1H)

stades b, c — Bêta-(thiényl-2), N-vinylphosphoramidate de diéthyle

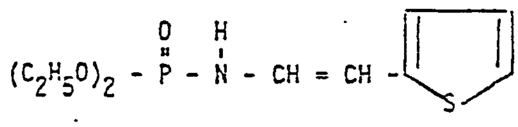

A une suspension de 11,2 g (0,1 mole) de terbutylate de potassium dans 160 ml de THF*, on ajoute goutte à goutte une solution de 27,9 g (0,1 mole) de N(thénylidène-2) aminométhylphosphonate de diéthyle dans 40 ml de THF*. Au cours de l'addition, la température s'élève de 20 à 35 °C. A la fin de l'addition, on porte la température à 40-45 °C pendant 30 mn puis le milieu réactionnel est versé sur 400 ml d'une solution aqueuse saturée de chlorure d'ammonium. La phase aqueuse est extraite à l'éther isopropylique puis les phases éthérées réunies sont lavées avec une solution saturée de chlorure de sodium, séchées sur sulfate de sodium et évaporées pour fournir 20,9 g (75 %) de produit référencé sous forme d'une huile jaune.

RMN ($CDCL_3$) δ/TMS
- 1,3 ppm (t, 6H)
- 3,95 ppm (ddeq, 4H)
- 6,35 ppm (m, 1H)
- 6,9 à 7,5 ppm (m, 5H) après échange avec $D_2O$ (m, 4H).

IR (film)
- NH 3 300 $cm^{-1}$
- C = C 1 650 $cm^{-1}$
- P—O 1 250 $cm^{-1}$
- P—O—C 1 050 $cm^{-1}$

stade d — N-[(thiényl-2)-2 éthyl], phosphoramidate de diéthyle

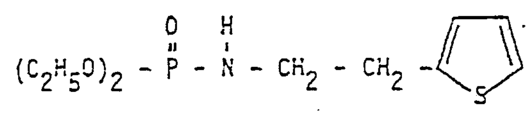

Les 20,9 g (0,075 mole) de bêta-(thiényl-2), N-vinylphosphoramidate de diéthyle obtenus ci-dessus sont additionnés à une solution de 2,85 g (0,075 mole) de borohydrure de sodium dans 200 ml d'éthanol.

Durant l'addition, la température s'élève puis se stabilise à 30 °C. Après 2 heures d'agitation supplémentaire, la température du milieu est portée à 45-50 °C pendant une heure, puis l'éthanol est évaporé et le résidu repris par un mélange d'éther isopropylique et d'eau.

La phase aqueuse est réextraite plusieurs fois à l'éther isopropylique et les phases organiques réunies sont lavées à l'eau, séchées sur sulfate de sodium et évaporées pour fournir 21 g (env. 75 % par rapport à l'aminométhylphosphonate de départ) de produit référencé sous forme d'une huile jaune.

IR (film)
- 3 400 $cm^{-1}$
- 1 520 $cm^{-1}$
- 1 275 $cm^{-1}$
- 1 210 $cm^{-1}$

RMN ($CDCL_3$) δ/TMS
- 1,3 ppm (t, 6H)
- 3,1 ppm (m, 5H) Ar—$CH_2$ $CH_2$—NH (par échange avec $D_2O$, on obtient à 3,1 ppm (m, 4H)
- 4,05 ppm (ddeq, 4H)
- 6,75 à 7,2 ppm (m, 3H).

stade e — N-orthochlorobenzyl, N-[(thiényl-2)-2 éthyl], phosphoramidate de diéthyle

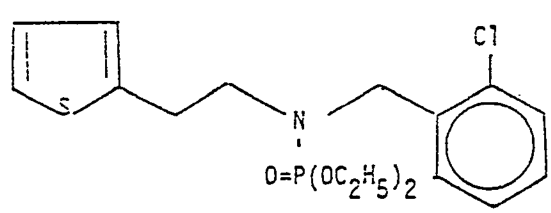

* THF ou tétrahydrofurane.

A une suspension de 3,6 g (0,075 mole) d'hydrure de sodium (à 50 % dans l'huile) dans 150 ml de toluène, on ajoute goutte à goutte sous agitation, à température ambiante, 21 g (0,075 mole) de N-[(thiényl-2)-2 éthyl] phosphoramidate de diéthyle dans 20 ml de toluène. Le milieu devenu rouge est porté durant 1 H à 80 °C puis additionné goutte à goutte de 16,1 g (0,1 mole) de chlorure d'orthochlorobenzyle. On maintient sous agitation durant 3H à 80 °C puis, après refroidissement, on lave à l'eau. La phase toluénique est ensuite évaporée pour fournir le phosphoramidate référencé (souillé de l'excès de chlorure d'orthochlorobenzyle) sous forme d'une huile utilisée telle quelle dans le stade suivant.

Un échantillon pur a été obtenu par chromatographie sur colonne de silice (éluant acétate d'éthyle).

IR (film)
- 3 000 $cm^{-1}$
- 1 550 $cm^{-1}$
- 1 250 $cm^{-1}$
- 1 050 $cm^{-1}$

RMN ($CDCl_3$) δ/TMS
- 1,3 ppm (t, 6H)
- 3,1 ppm (m, 4H)
- 4 ppm (ddeq, 4H)
- 4,45 ppm (d, 2H)
- 6,8 à 7,6 ppm (m, 7H)

stade f — Chlorhydrate de N-orthochlorobenzyl, (thiényl-2)-2 éthylamine

Le phosphoramidate brut obtenu ci-dessus est traité par 200 ml d'une solution aqueuse d'acide chlorhydrique 3N durant 2 H à 90 °C. Après extraction à cette température par 2 × 20 ml de dichloro 1,2 éthane et refroidissement de la solution aqueuse, il se forme un précipité que l'on filtre. On obtient ainsi, après séchage, 15 g (52 % par rapport à l'aminométhylphosphonate de diéthyle) de chlorhydrate référencé sous forme de cristaux blancs.

F = 143 °C

IR (pastilles KBr)
- 3 400 $cm^{-1}$
- 2 900 à 2 600 $cm^{-1}$
- 1 575 $cm^{-1}$
- 1 450 $cm^{-1}$

RMN (DMSO $d_6$) δ/TMS
- 7 à 7,6 ppm (m, 8H)
- 3,35 ppm (s, 4H)
- 4,15 ppm (s, 2H)
- env. 10 ppm (m, 2H) échangeable avec $D_2O$

Analyse : $C_{13}H_{14}Cl$ NS, HCl (M = 288,236)
Calculé : C 54,16 H 5,24 N 4,85 %
Trouvé : C 54,11 H 5,28 N 4,80 %

## Exemple 2

Préparation du chlorhydrate de N-orthochlorobenzyl (thiényl-2)-2 éthylamine

Variante B

stade a — N(thénylidène-2), aminométhylphosphate de diéthyle

On prépare 0,1 mole du produit référencé selon le procédé décrit à l'exemple 1.

stades b, d bis — N-orthochlorobenzyl, N-[bêta-(thiényl-2)vinyl], phosphoramidate de diéthyle

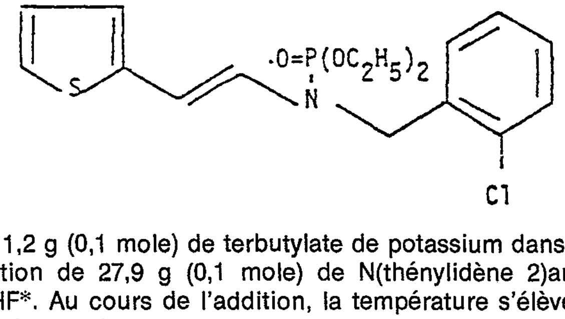

A une suspension de 11,2 g (0,1 mole) de terbutylate de potassium dans 160 ml de THF* ; on ajoute goutte à goutte une solution de 27,9 g (0,1 mole) de N(thénylidène 2)aminométhylphosphonate de diéthyle dans 40 ml de THF*. Au cours de l'addition, la température s'élève de 20 à 35 °C. A la fin de l'addition, on porte la température à 40-45 °C pendant 30 mn, puis on additionne goutte à goutte 16,1 g (0,1 mole) de chlorure d'orthochlorobenzyle. L'addition terminée, on porte le milieu au reflux pendant 3 H

* THF ou tétrahydrofurane.

puis on évapore le THF*. Le résidu est repris par de l'éther isopropylique et la phase éthérée, lavée à l'eau, séchée puis évaporée, fournit 32,7 g (85 %) de produit référencé sous forme d'une huile jaune orangé que l'on utilise telle quelle dans le stade suivant.

Un échantillon purifié par chromatographie sur colonne de silice (éluant : acétate d'éthyle) présente les caractéristiques suivantes :

IR (film)
- $3\,000\ cm^{-1}$
- $1\,650\ cm^{-1}$
- $1\,240\ cm^{-1}$
- $1\,045\ cm^{-1}$

RMN ($CDCl_3$) δ/TMS
- 1,3 ppm (t, 6H)
- 4,05 ppm (m, 6H)
- 6,7 à 7,5 ppm (m, 7H).

stade e bis — N-orthochlorobenzyl N-[(thiényl-2)-2 éthyl] phosphoramidate de diéthyle

Le produit brut obtenu dans l'étape précédente est ajouté à une suspension de 6,5 g (0,17 mole) de borohydrure de sodium dans 100 ml de dioxanne. Au milieu réactionnel, refroidi à 0 °C, on additionne goutte à goutte 14,3 g (0,17 mole) d'acide trifluoracétique. L'addition terminée, on chauffe durant 1 H au reflux puis hydrolyse après refroidissement par addition de 200 ml d'eau. Le milieu est extrait au chlorure de méthylène et la phase organique isolée, séchée sur sulfate de sodium et évaporée, fournit 33 g (85 % par rapport à l'aminométhylphosphate de départ) de phosphoramidate référencé sous forme d'une huile jaune. Un échantillon purifié par chromatographie sur colonne de silice présente des caractéristiques identiques à celui obtenu par la variante A.

stade f — Chlorhydrate de N-orthochlorobenzyl, (thiényl-2)-2 éthylamine

En opérant comme décrit à l'exemple 1, on obtient 17,3 g (rendement 60 % par rapport à l'aminométhylphosphonate de diéthyle) de chlorhydrate de N-orthochlorobenzyl, (thiényl-2)-2 éthylamine dont les caractéristiques physiques, spectrales et analytiques sont identiques à celles du produit préparé à l'exemple 1.

## Revendications

1. Procédé de préparation des dérivés de (thiényl-2)- et (thiényl-3)-2 éthylamines de formule

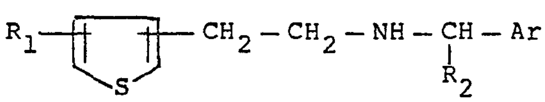

(I)

dans laquelle :

$R_1$ (en position 2, 3, 4 ou 5) représente un atome d'hydrogène, un radical alkyle linéaire ou ramifié, un radical aromatique hétérocyclique ou non éventuellement mono- ou polysubstitué par des groupes alkyle, phényle, halogène, nitro, cyano, amino, carboxy ou alcoxy, ou bien un radical alcoxy, un atome d'halogène, un radical nitro, carboxyle, cyano ou amino ;

dans la chaîne aminoéthyle, qui peut occuper les positions 2 ou 3 du noyau thiophénique, $R_2$ signifie un atome d'hydrogène ou un radical alkyle linéaire ou ramifié, un radical aromatique hétérocyclique ou non éventuellement mono- ou polysubstitué par des groupes alkyle, phényle, halogène, nitro, cyano, amino, carboxy ou alcoxy ;

Ar représente un radical aromatique hétérocyclique ou non éventuellement mono- ou polysubstitué par des groupes alkyle, phényle, halogène, nitro, cyano, amino, carboxy ou alcoxy, caractérisé en ce qu'on condense un dérivé de formule II

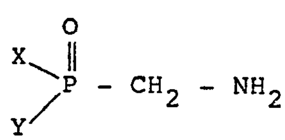

(II)

dans laquelle X et Y sont identiques ou différents et représentent un radical alkyle, aryle, alcoxy, dialkyl- ou diaryl-amino, avec un composé carbonylé de formule III

* THF ou tétrahydrofurane.

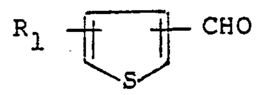

(III)

dans laquelle $R_1$ est tel que défini pour la formule I, pour conduire à un composé de formule IV

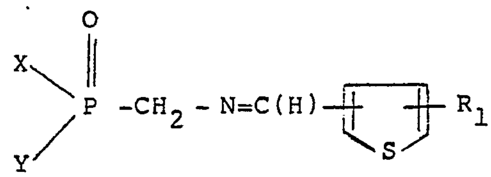

(IV)

dans laquelle $R_1$, X et Y ont les significations fournies précédemment, on traite le composé de formule IV avec une base de formule $B^{\ominus}M^{\oplus}$ dans laquelle $M^{\oplus}$ est un métal alcalin ou alcalino-terreux ou un organomagnésium et $B^{\ominus}$ est un carbanion choisi parmi les groupes chargés négativement hydrogène, hydroxy, amidure, mono- ou dialkylamidure, alkyle linéaire ou ramifié, ou alcoxy linéaire ou ramifié, pour obtenir un carbanion de formule V

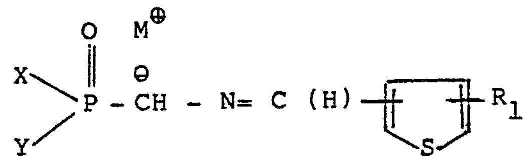

(V)

dans laquelle $R_1$, X, Y et $M^{\oplus}$ ont les significations précédentes, qui se transforme à des températures comprises entre —78 °C et + 150 °C en dérivé VI

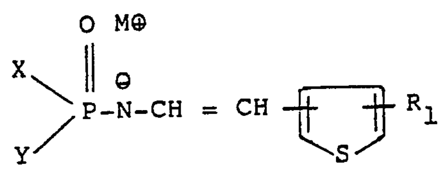

(VI)

dans laquelle $R_1$, X, Y et $M^{\oplus}$ ont les significations précédentes, pour aboutir, après reprise par l'eau, au composé de formule VII

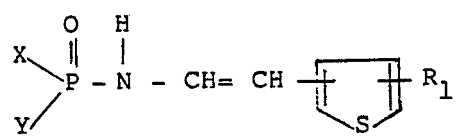

(VII)

dans laquelle $R_1$, X et Y ont les significations précédentes, que l'on transforme sous l'action d'un agent réducteur en un dérivé de formule VIII

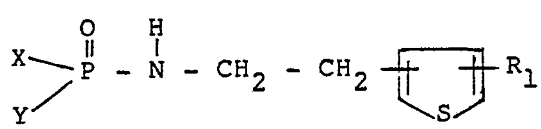

(VIII)

dans laquelle $R_1$, X et Y ont les significations précédentes, que l'on met à réagir successivement avec une base de formule $B'^{\ominus}M'^{\oplus}$, dans laquelle $B'^{\ominus}$ et $M'^{\oplus}$ ont respectivement les mêmes significations de $B^{\ominus}$ et $M^{\oplus}$ ci-dessus, puis avec un dérivé halogène de formule IX

$$Ar—CHX'—R_2 \quad (IX)$$

dans laquelle Ar et $R_2$ sont tels que définis pour la formule I et X' représente un halogène, pour obtenir un dérivé de formule (X)

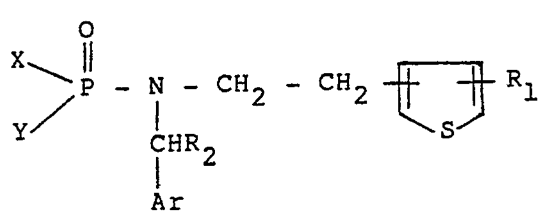

(X)

dans laquelle $R_1$, $R_2$, X, Y et Ar ont les significations précédentes, qui est finalement transformé sous l'action d'un acide en composé de formule I.

2. Procédé selon la revendication 1, caractérisé en ce que Ar est un groupe thiényle, furyle, pyridyle, phényle ou naphtyle.

3. Procédé selon la revendication 1, caractérisé en ce que M⊕ ou M'⊕ est un métal choisi parmi le sodium, le lithium, le potassium et le magnésium.

4. Procédé suivant la revendication 1, caractérisé en ce que, selon une variante à condition d'utiliser une quantité stœchiométrique de la base B⊖M⊕, le dérivé de formule VI est mis à réagir avec le dérivé halogéné de formule IX puis réduit pour obtenir directement le dérivé de formule X.

5. Procédé suivant la revendication 1, caractérisé en ce que la réaction de transformation en dérivé (V), en dérivé (VI) s'effectue dans un solvant organique choisi parmi des éthers linéaires ou cycliques, des hydrocarbures aromatiques, des alcools, des amides ou des sulfoxydes.

6. Procédé selon la revendication 5, caractérisé en ce que le solvant choisi parmi le tétrahydrofuranne, le benzène, le toluène, les xylènes, le diméthylformamide et le diméthylsulfoxyde.

7. Procédé suivant la revendication 1, caractérisé en ce que la réduction du dérivé de formule VII est effectuée par un borohydrure de métal alcalin, notamment le borohydrure de sodium ou le borohydrure de potassium.

## Claims

1. Process for the preparation of 2-(2-thienyl)- and -(3-thienyl) ethylamine derivatives of the formula (I)

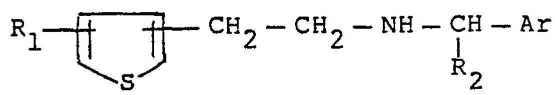

(I)

in which :

$R_1$ (at 2-, 3-, 4- or 5-position) represents a hydrogen atom, a straight- or branched-chain alkyl radical, a heterocyclic or non-heterocyclic aromatic radical, optionally mono- or poly-substituted with an alkyl, phenyl, halo, nitro, cyano, amino, carboxy or alkoxy group ; or an alkoxy radical, a halogen atom, a nitro, carboxyl, cyano or amino radical ;

in the aminoethyl chain which may occupy positions 2 or 3 of the thiophene nucleus, $R_2$ represents a hydrogen atom or a straight- or branched-chain alkyl radical, a heterocyclic or non-heterocyclic aromatic radical, optionally mono- or poly-substituted with an alkyl, phenyl halo, nitro, cyano, amino, carboxy or alkoxy group ;

Ar represents a heterocyclic or non-heterocyclic aromatic radical, optionally mono- or poly-substituted with an alkyl, phenyl, halo, nitro, cyano, amino, carboxy or alkoxy group, comprising : condensing a derivative of the formula (II) :

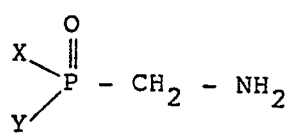

(II)

in which X and Y represent independently from each other an alkyl, aryl, alkoxy, aryloxy, dialkyl- or diaryl-amino radical with a carbonyl compound of the formula (III) :

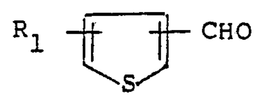

(III)

in which $R_1$ is as defined for formula (I), to give a compound of the formula (IV) :

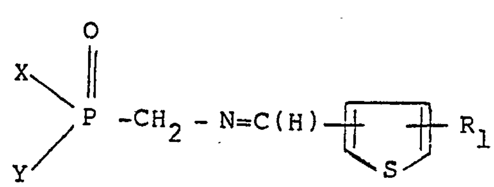

(IV)

in which $R_1$, X and Y are previously defined ; treating the compound of the formula (IV) with a base of the

formula B⊖M⊕, wherein M⊕ is an alcali or alcaline earth metal or an organomagnesium and B⊖ is a carbanion selected from the negatively charged group hydrogen, hydroxy, amide, mono- or dialkylamide, straight- or branched-chain alkyl or straight- or branched-chain alkoxy, to obtain a carbanion of formula (V),

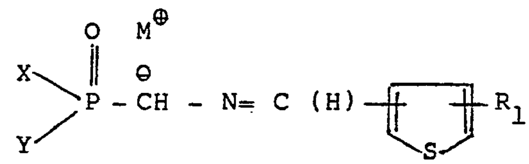

(V)

in which $R_1$, X, Y and M+ are as previously defined, which is converted at temperatures between —78 °C and + 150 °C into derivative (VI) :

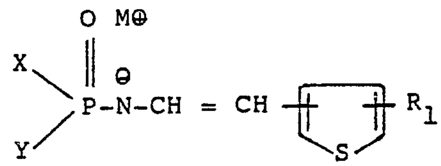

(VI)

in which $R_1$, X, Y and M+ are as previously defined, to give, after taking up with water, compound (VII) :

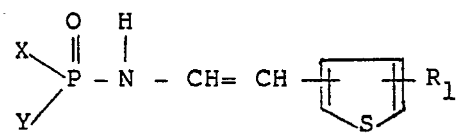

(VII)

in which $R_1$, X and Y are as previously defined, which is converted, under the action of a reducing agent, to a derivative of the formula (VIII) :

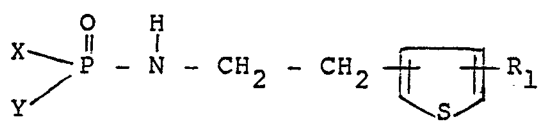

(VIII)

in which $R_1$, X and Y are as previously defined, which is reacted successively with a base of the formula B'⊖M'⊕, in which B'⊖ and M'⊕ are respectively as B⊖ and M⊕ above, and then with a halogenated derivative of the formula (IX) :

$$Ar—CHX'—R_2 \qquad (IX)$$

in which Ar and $R_2$ are as defined in formula (I) and X' is halogen, to give a derivative of the formula (X) :

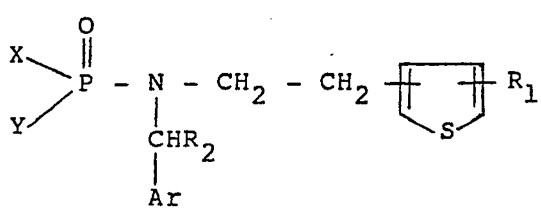

(X)

in which $R_1$, $R_2$, X, Y and $A_r$ are as above defined, and which is converted, under the action of an acid, to a compound of the formula (I).

2. Process as claimed in claim 1, wherein Ar is a thienyl, furyl, pyridyl, phenyl or naphthyl group.

3. Process as claimed in claim 1, wherein M⊕ or M'⊕ is a metal selected from sodium, lithium, potassium and magnesium.

4. Process as claimed in claim 1, wherein, according to another embodiment, with the proviso that base B⊖M⊕ is used in a stoichiometric amount, the derivative of the formula (VI) is reacted with the halogenated derivative of the formula (IX) and is then reduced, to give directly the derivative of the formula (X).

5. Process as claimed in claim 6 or 7, wherein the conversion reaction of derivative (V) into derivative

(VI) is carried out within an organic solvent selected from straight-chain and cyclic ethers, aromatic hydrocarbons, alcohols, amides and sulfoxides.

6. Process as claimed in claim 5, wherein the solvent is selected from tetrahydrofuran, benzene, toluene, xylenes, dimethylformamide, and dimethylsulfoxide.

7. Process as claimed in claim 1, wherein the reduction of the derivative of the formula (VII) is effected with an alkali metal borohydride, typically sodium borohydride or potassium borohydride.

**Patentansprüche**

1. Verfahren zur Herstellung von 2-(2-Thienyl)- und (3-Thienyl)-ethylaminderivaten der Formel

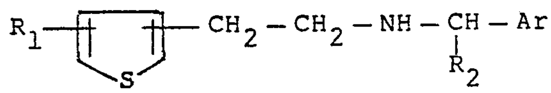
(I)

in der

$R_1$ (in Position 2, 3, 4, oder 5) ein Wasserstoffatom, einen unverzweigten oder verzweigten Alkylrest, eine aromatischen heterocyclischen oder nichtheterocyclischen Rest, der gegebenenfalls mittels Alkyl-, Phenyl-, Halogen-, Nitro-, Cyano-, Amino-, Carboxy- oder Alkoxygruppen mono- oder polysubstituiert ist, oder auch einen Alkoxyrest, ein Halogenatom, einen Nitro-, Carboxyl-, Cyano- oder Aminorest darstellt ;

$R_2$ in der Aminoethylkette, die die Positionen 2 oder 3 des Thiophenkernes einnehmen kann, ein Wasserstoffatom oder einen unverzweigten oder verzweigten Alkylrest, einen aromatischen heterocyclischen oder nichtheterocyclischen Rest bezeichnet, der gegebenenfalls mittels Alkyl-, Phenyl-, Halogen-, Nitro-, Cyano-, Amino-, Carboxy- oder Alkoxygruppen mono- oder polysubstituiert ist ;

Ar einen aromatischen heterocyclischen oder nichtheterocyclischen Rest darstellt, der gegebenenfalls mittels Alkyl-, Phenyl-, Halogen-, Nitro-, Cyano-, Amino-, Carboxy- oder Alkoxygruppen mono- oder polysubstituiert ist, dadurch gekennzeichnet, daß man ein Derivat der Formel (II)

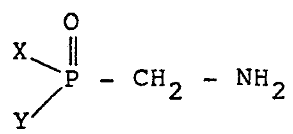
(II)

in der X und Y identisch oder verschieden sind und einen Alkyl-, Aryl-, Alkoxy-, Dialkyll- oder Diaryl-Amino-Rest darstellen, mit einer Carbonylverbindung der Formel (III) kondensiert

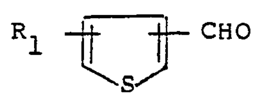
(III)

in der $R_1$ wie in Formel (I) definiert ist, um zu einer Verbindung der Formel (IV) zu gelangen

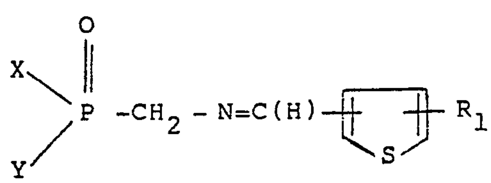
(IV)

in der $R_1$, X und Y die oben genannten Bedeutungen haben, man die Verbindung der Formel (IV) mit einer Base der Formel $B^-M^+$ behandelt, in der $M^+$ ein Alkali- oder Erdalkalimetall oder eine Organomagnesiumverbindung ist und $B^-$ ein Carbanion ist, ausgewählt aus den negativ geladenen Gruppen Wasserstoff, Hydroxy, Amid, Mono- oder Dialkylamid, unverzweigtes oder verzweigtes Alkyl, oder unverzweigtes oder verzweigtes Alkoxy, um ein Carbanion der Formel (V) zu erhalten

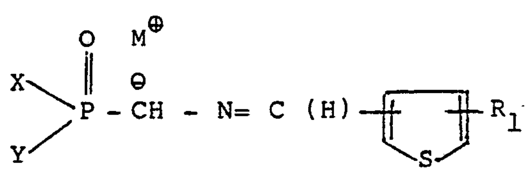
(V)

in der $R_1$, X, Y und $M^+$ die vorhergehenden Bedeutungen haben, das sich bei Temperaturen zwischen —78 °C und + 150 °C in das Derivat VI umformt

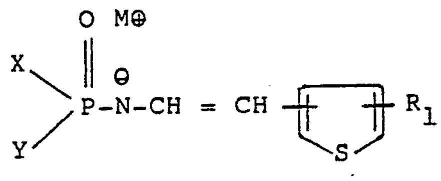

(VI)

in der $R_1$, X, Y und $M^+$ die vorhergehenden Bedeutungen haben, um, nach der Wiederaufnahme mit Wasser, zu der Verbindung der Formel (VII) zu führen

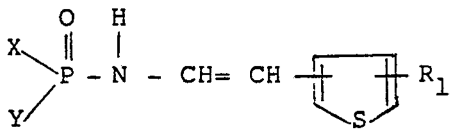

(VII)

in der $R_1$, X und Y die vorhergehenden Bedeutungen haben, die man unter Einwirkung eines Reduktionsmittels in ein Derivat der Formel (VIII) umformt

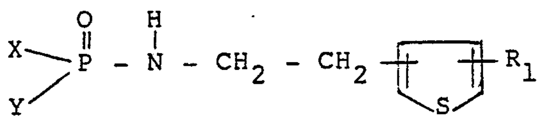

(VIII)

in der $R_1$, X und Y die vorhergehenden Bedeutungen haben, das man nach und nach mit einer Base der Formel $B'^-M'^+$, in der $B'^-$ und $M'^+$ respektiv dieselben Bedeutungen wie oben $B^-$ und $M^+$ haben, und anschließend mit einem Halogenderivat der Formel (IX) reagieren läßt

$$AR—CHX'—R_2 \qquad (IX)$$

in der Ar und $R_2$ wie in Formel (I) definiert sind und X' ein Halogen darstellt, um ein Derivat der Formel (X) zu erhalten

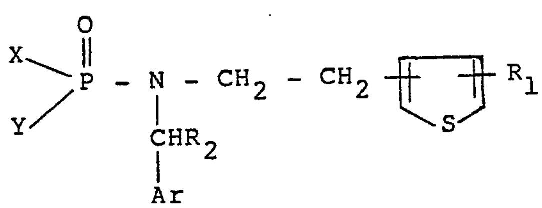

(X)

in der $R_1$, $R_2$, X, Y und Ar die vorhergehenden Bedeutungen haben, die schließlich unter Einwirkung einer Säure in die Verbindung der Formel (I) überführt wird.

2. Verfahren gemäß Anspruch 1, dadurch gekennzeichnet, daß Ar eine Thienyl-, Furyl-, Pyridyl-, Phenyl- oder Naphthylgruppe ist.

3. Verfahren gemäß Anspruch 1, dadurch gekennzeichnet, daß $M^+$ oder $M'^+$ ein Metall ist, ausgewählt aus Natrium, Lithium, Kalium und Magnesium.

4. Verfahren gemäß Anspruch 1, dadurch gekennzeichnet, daß gemäß einer Variante unter Benutzung einer stöchiometrischen Menge der Base $B^-M+$ das Derivat der Formel (VI) mit dem Halogenderivat der Formel (IX) zur Reaktion gebracht wird und anschließend reduziert wird, um direkt das Derivat der Formel (X) zu erhalten.

5. Verfahren gemäß Anspruch 1, dadurch gekennzeichnet, daß die Transformationsreaktion in Derivat (V) und Derivat (VI) in einem organischen Lösungsmittel bewirkt wird, das ausgewählt ist aus linearen und cyclischen Ethern, aromatischen Kohlenwasserstoffen, Alkoholen, Amiden oder Sulfoxiden.

6. Verfahren nach Anspruch 5, dadurch gekennzeichnet, daß das Lösungsmitel ausgewählt ist aus Tetrahydrofuran, Benzol-Toluol, Xylol, Dimethylformamid und Dimethylsulfoxid.

7. Verfahren gemäß Anspruch 1, dadurch gekennzeichnet, daß die Reduktion des Derivates der Formel (VII) mittels eines Alkalimetallborhydrides bewirkt wird, insbesondere Natriumborhydrid oder Kaliumborhydrid.